(19) Europäisches Patentamt
European Patent Office
Office européen des brevets

(11) Veröffentlichungsnummer: **0 060 808**
**B1**

(12) # EUROPÄISCHE PATENTSCHRIFT

(45) Veröffentlichungstag der Patentschrift: **24.07.85**

(51) Int. Cl.[4]: **C 07 D 305/12**

(21) Anmeldenummer: **82810107.1**

(22) Anmeldetag: **11.03.82**

(54) **4-Chlor-4-chlormethyloxetan-2-on und Verfahren zu dessen Herstellung.**

(30) Priorität: **17.03.81 GB 8108236**

(43) Veröffentlichungstag der Anmeldung: **22.09.82 Patentblatt 82/38**

(45) Bekanntmachung des Hinweises auf die Patenterteilung: **24.07.85 Patentblatt 85/30**

(84) Benannte Vertragsstaaten: **BE CH DE FR GB IT LI**

(56) Entgegenhaltungen:
**JOURNAL OF THE AMERICAN CHEMICAL SOCIETY, Band 62, 1940, Easton C.D. HURD et al. "The Chlorination and the Structure of Acetylketene" Seiten 1147 bis 1148**

(73) Patentinhaber: **CIBA-GEIGY AG, Postfach, CH-4002 Basel (CH)**

(72) Erfinder: **Dingwall, John Grey, Dr., Niederholzstrasse 51, CH-4125 Riehen (CH)**
Erfinder: **Tuck, Brian, Dr., 91, Seal Road Bramhall, Stockport Cheshire SK7 2LE (GB)**

**EP 0 060 808 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 4-Chlor-4-chlormethyloxetan-2-on als neue Verbindung und ein Verfahren zu dessen Herstellung.

Das erfindungsgemässe 4-Chlor-4-chlormethyloxetan-2-on entspricht der Formel I

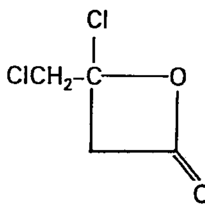

(I).

Es ist bekannt, dass die Chlorierung von Diketen mit molekularem Chlor $ClCH_2COCH_2COCl$ ergibt:

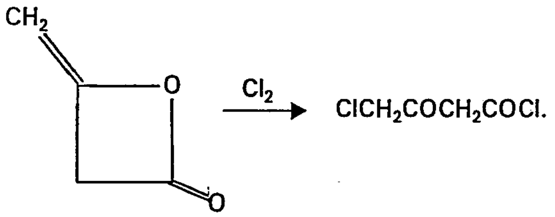


Bevor die genaue Struktur von Diketen abgeklärt worden war, stellten Hurd und Abernethy 1940 [JACS, 62, 1147 (1940)] die These auf, das 4-Chlor-4-chlormethyloxetan-2-on könnte bei der Herstellung von $ClCH_2COCH_2COCl$ durch Chlorierung von Diketen mit Chlor in Tetrachlorkohlenstoff als Zwischenprodukt entstehen. Auf Grund anderer Beweise wurde diese Hypothese von den Autoren jedoch wieder verworfen.

Erfindungsgemäss wird das 4-Chlor-4-chlormethyloxetan-2-on durch freie Radikalchlorierung von Diketen mit Sulfurylchlorid hergestellt. Die Verbindung ist eine stabile destillierbare Flüssigkeit. Ferner wurde festgestellt, dass sie unter den von Hurd und Abernethy für die Chlorierung von Diketen angewandten Bedingungen, d.h. bei der Umsetzung von Diketen mit Chlor bei 0°C in Tetrachlorkohlenstofflösung, nicht reaktiv ist. Die Möglichkeit, dass bei der ionischen Chlorierung von Diketen zum $\gamma$-Chloracetoacetylchlorid das 4-Chlor-4-chlormethyloxetan-2-on als Zwischenprodukt entsteht, ist daher sowohl vom theoretischen als auch praktischen Standpunkt aus vollständig zu verneinen.

Gegenstand der Erfindung ist auch ein Verfahren zur Herstellung von 4-Chlor-4-chlormethyloxetan-2-on durch Umsetzung von Diketen mit Sulfurylchlorid unter Bedingungen, bei denen freie Radikale gebildet werden.

Die Umsetzung kann so durchgeführt werden, dass die freien Radikale durch ionisierende oder Ultraviolett-Bestrahlung des Reaktionsgemisches gebildet werden. Die Umsetzung kann aber auch in Gegenwart einer freie Radikale bildenden chemischen Substanz vorgenommen werden. Als Beispiele seien genannt:

i) organische Peroxide, wie z.B. tert-Butylperacetat, tert-Butylperbenzoat, Acetylperoxid, Benzoylperoxid, Di-isopropylperdicarbonat, Di-tert-butylcyclohexylperdicarbonat, Di-tert-butylperoxid, tert-Butylhydroperoxid;

ii) anorganische Peroxy-Verbindungen, wie z.B. Wasserstoffperoxid und Ammoniumpersulfat;

iii) organische Azoverbindungen, wie z.B. Azobisisobutyronitril und Azobisisopropan;

iv) eine Kombination von (i) oder (iii) mit UV-Bestrahlung;

v) eine Kombinaton von (i) oder (ii) mit einem Metallionen-Katalysator, z.B. Cu, V, Fe und Ti, die radikalbildende Redox-Systeme ergeben, z.B.

$H_2O_2 + Fe^{2+}$

$NH_4^{+-}O_3S\text{-}O\text{-}J\text{-}SO_3^{-}NH_4^{+} + Cu^{+}$

tert-Butylhydroperoxid + $Ti^{2+}$.

Die freie Radikale bildenden Verbindungen werden bevorzugt in katalytischen Mengen, z.B. von 0,1 bis 10 Mol.%, insbesondere von 1 bis 5 Mol.%, bezogen auf eingesetzte Diketenmenge, verwendet. Gewünschtenfalls kann die Umsetzung in Gegenwart eines unter den Reaktionsbedingungen inerten Lösungmittels vorgenommen werden. Beispiele geeigneter inerter Lösungsmittel sind Petroläther, Benzol, Chlorbenzol und vorzugsweise Tetrachlorkohlenstoff. Die Reaktionstemperatur hängt von der zur Radikalbildung verwendeten Methode ab. Im allgemeinen wird die Umsetzung jedoch zweckmässig bei Temperaturen zwischen 0 und 100°C durchgeführt. Die Umsetzung kann in einzelnen Chargen oder kontinuierlich, z.B. in einem Kaskadenreaktor, vorgenommen werden.

Die Verbindung der Formel I zeigt im Bereich bis zu 1000 $\mu$g/ml antimikrobielle Wirkung gegen verschiedene Organismen. Mit niedrigen Konzentrationen an Verbindung der Formel I kann vor allem das Wachstum von Bakterien und Pilzen, z.B. Staphylococcus aureus, Escherichia coli, Salmonella typhi, Proteus vulgaris, Pseudomonas aeruginosa, Candida albicans and Aspergillus niger, bekämpft werden. Die Verbindung der Formel I kann bei der Verwendung als antimikrobielles Mittel in reiner Form oder als konzentrierte oder verdünnte Lösung in einem mit wässrigen Systemen mischbaren oder nicht-mischbaren inerten Lösungsmittel eingesetzt werden. Beispiele geeigneter inerter Lösungsmittel sind Kohlenwasserstoffe, z.B. Kerosen; aromatische Lösungsmittel, wie Xylol; Ketone, z.B. Aceton; oder Glykoläther, z.B. 1,2-Dimethoxyäthan, 1,2-Diäthoxyäthan, Diäthylenglykoldimethyläther und Diäthylenglykoldiäthyläther. Derartige Lösungen können gegebenenfalls Emulgatoren oder Dispergiermittel enthalten oder sie können mit Alkalimetall- und Erdalkalimetallcarbonaten stabilisiert sein. Die Verbindung der Formel I kann auch im Gemisch mit festen Trägerstoffen verwendet werden. Gewünschtenfalls kann die Verbindung der Formel I zusammen mit anderen antimikrobiellen Wirkstoffen eingesetzt werden.

Die Verbindung der Formel I ist ferner ein wertvolles Zwischenprodukt zur Herstellung von $\gamma$-Chloracetessigsäurederivaten. Bekanntlich

können γ-Chloracetessigsäurederivate, z.B. Ester oder Arylamide, aus γ-Chloracetoacetylchlorid hergestellt werden und sind Zwischenprodukte für z.B. Pigmente oder Farbstoffe.

Beispiele für die Verwendung von γ-Chloracetessigester für die Herstellung von Pigmenten finden sich in Liebigs Annalgen, 313, 12-13 (1900) und in der schweizerischen Patentschrift 582.218, wonach γ-Chloracetessigester mit diazotierten aromatischen Aminen zu den entsprechenden Arylhydrazonderivaten umgesetzt werden. Diese letzteren ergeben beim Erhitzen mit wässrigem Alkali unter Cyclisierung und Hydrolyse Pyrazoloncarbonsäuren der Formel II

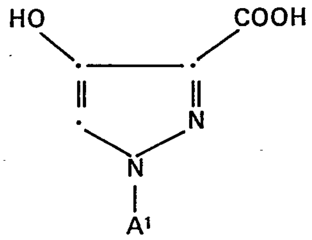

(II).

Durch Umsetzung von Verbindungen der Formel II mit aromatischen Diazoniumsalzen, anschliessende Überführung in das Säurechlorid und weitere Umsetzung mit Diaminen werden Pigmente der Formel III

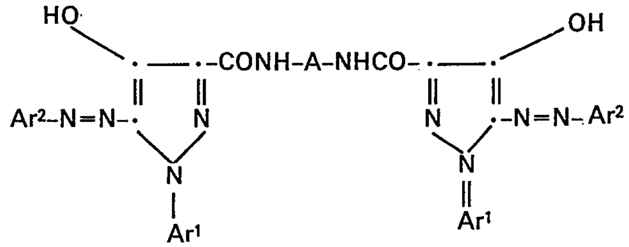

(III)

erhalten, wobei $Ar^1$ und $Ar^2$ aromatische Reste und A einen Kohlenwasserstoffrest oder die direkte Bindung darstellen. γ-Chloracetoacetylchlorid kann jedoch nicht leicht gereinigt werden und muss wegen der ungenügenden Lagerstabilität rasch verwendet werden. Demgegenüber lässt sich die Verbindung der Formel I leicht reinigen und ist lagerstabil; sie stellt somit für die Herstellung der oben genannten γ-Chloracetessigester und -arylamide ein günstigeres Zwischenprodukt als das γ-Chloracetylchlorid dar.

In den folgenden Beispielen bedeuten Teile und Prozente Gewichtsteile bzw. Gewichtsprozente.

Beispiel 1

2,5 Teile Benzoylchlorid, das 25% Wasser enthält, werden in 408 Teilen Tetrachlorkohlenstoff suspendiert. Das Gemisch wird gerührt, um das Benzoylperoxid zu lösen, und dann über Magnesiumsulfat getrocknet. Zu dieser Lösung gibt man 67,5 Teile Sulfurylchlorid, und die erhaltene Lösung tropft man innerhalb 1,5 Stunden zu einer rückflussierenden Lösung von 42 Teilen Diketen in 816 Teilen Tetrachlorkohlenstoff. Nach Beendigung der Zugabe wird das Gemisch unter Rückfluss noch während 1,5 Stunden erhitzt, und dann wird der Tetrachlorkohlenstoff am Wasserstrahlvakuum entfernt. Die Destillation des Rückstandes bei 0,01 Millibar ergibt 4-Chlor-4-chlormethyloxetan-2-on; Sdp. 35-36°C, das beim Abkühlen unter 20°C kristallisiert.

$IR\gamma_{max}$ (C=O) 1850 $cm^{-1}$; $^1H$-NMR ($CCl_4$): $\delta = 4{,}14$ (s, 2H, Cl-$CH_2$);
3,82 und 4,20 (beide d, 1H, J = 17Hz, $H_2C(-3)$).

Analyse für $C_4H_4Cl_2O_2$

| | | | |
|---|---|---|---|
| ber. | C 31,00 | H 2,60 | Cl 45,75 |
| gef. | C 30,51 | H 2,51 | Cl 45,45. |

Bei 0°C wird Chlor in eine Lösung von 4-Chlor-4-chlormethyloxetan-2-on in Tetrachlorkohlenstoff eingeleitet. Die so erhaltene Lösung lässt man mehrere Stunden stehen. Nach dem Entfernen des Tetrachlorkohlenstoffs und des Chlors erhält man unverändertes 4-Chlor-4-chlormethyloxäthan-2-on.

Beispiel 2

Ein Gemisch aus 13,5 Teilen Sulfurylchlorid, 8,4 Teilen Diketen, 0,5 Teilen trockenem Benzoylperoxid und 720 Teilen Tetrachlorkohlenstoff wird bei Raumtemperatur 5 Stunden mit UV-Licht bestrahlt. Das Lösungsmittel wird unter vermindertem Druck entfernt, und der Rückstand wird in einem Kugelrohr bei 50°C und einem Druck von 0,03 Millibar destilliert. Man erhält das mit der Verbindung gemäss Beispiel 1 identische 4-Chlor-4-chlormethyloxetan-2-on in Form eines farblosen Öls.

Beispiel 3

Ein Gemisch von 42,0 Teilen Diketen und 6,5 Teilen tert-Butylcyclohexylperdicarbonat, das in 400 Teilen Tetrachlorkohlenstoff gelöst ist, wird innerhalb von 30 Minuten zu einem rückflussierenden Gemisch von 67,5 Teilen Sulfurylchlorid und 800 Teilen Tetrachlorkohlenstoff zugetropft. Nach Beendigung der Zugabe wird das Gemisch noch während 30 Minuten am Rückfluss gehalten. Anschliessend wird der Tetrachlorkohlenstoff unter vermindertem Druck entfernt. Die Destillation des Rückstandes bei 0,03 Millibar ergibt 4-Chlor-4-chlormethyloxetan-2-on, Sdp. 42-46°C, das mit der Verbindung gemäss Beispiel 1 identisch ist.

Beispiel 4

Eine Lösung von Tetrachlorkohlenstoff, die 42 Teile Diketen, 6 Teile Bis-(tert-butylcyclohexyl)-perdicarbonat und 67,5 Teile Sulfurylchlorid enthält, wird so durch einen Zwei-Gefäss-Kaskadenreaktor gepumpt, dass eine Verweilzeit von 75 Minuten resultiert. Die Verweilzeit errechnet sich aus

$$\frac{\text{Volumen des Reaktionsgefässes}}{\text{pro Minute durchgepumptes Volumen.}}$$

Die Reaktionsgefässe werden kräftig gerührt und auf Rückflusstemperatur erhitzt. Der Produktestrom enthält 4-Chlor-4-chlormethyloxethan-2-on. Der Tetrachlorkohlenstoff wird an einem Rotationsverdampfer abdestilliert. Das Rohprodukt wird durch Destillation an einem Dünnschichtverdampfer gereinigt. Man erhält 4-Chlor-4-chlormethyloxetan-2-on, das mit der Verbindung gemäss Beispiel 1 identisch ist.

Beispiel 5

Eine gemischte Kultur von Pseudomonas aeruginosa, Enterobacter aerogenes, Escherichia coli, Proteus vulgaris, Bacillus mycoides und Staphylococcus aureus in einer Salzlösung, die $10^7$ Organismen/ml enthält, wird mit 30 ppm der Verbindung gemäss Beispiel 1 behandelt. Nach einer Stunde hat sich die Zahl der Organismen auf $10^2$ Organismen/ml reduziert, was die schnelle bakterizide Wirkung der Verbindung des Beispiels 1 beweist.

Verwendung von 4-Chlor-4-chlormethyloxetan-2-on als Zwischenprodukt zur Herstellung von γ-Chloracetessigsäurederivaten

Beispiel 6

7,8 Teile 4-Chlor-4-chlormethyloxetan-2-on werden in 200 Teilen Methanol, das 4,2 Teile wasserfreies Natriumbicarbonat enthält, gelöst. Nach 20 Stunden Rühren bei Raumtemperatur werden die Feststoffe abfiltriert, das Filtrat wird verdampft, und der Rückstand wird destilliert. Man erhält Methl-γ-chloracetoacetat; Sdp. 48–50°C/0,8 Millibar.

Beispiel 7

7,8 Teile 4-Chlor-4-chlormethyloxetan-2-on werden in 150 Teilen Methylenchlorid, das 4,2 Teile wasserfreies Natriumbicarbonat enthält, gelöst, und die Lösung wird auf 0°C abgekühlt. Dann gibt man innerhalb von 30 Minuten 4,7 Teile Anilin zu, und das Reaktionsgemisch wird noch 48 Stunden bei Raumtemperatur gerührt. Die Feststoffe werden abfiltriert, und das Filtrat wird verdampft. Der Rückstand wird zweimal aus Chloroform kristallisiert. Man erhält γChloracetoacetanilid; Smp. 142–3°C.

Beispiel 8

7,8 Teile 4-Chlor-4-chlormethyloxetan-2-on werden in 150 Teilen Methylenchlorid, das 4,2 Teile wasserfreies Natriumbicarbonat enthält, gelöst, und das Gemisch wird auf 0°C abgekühlt. 5,4 Teile p-Toluidin, gelöst in 50 Teilen Methylenchlorid, werden innerhalb von 30 Minuten hinzugegeben. Dann lässt man die Temperatur des Reaktionsgemisches auf Raumtemperatur ansteigen und rührt bei dieser Temperatur 48 Stunden. Die Feststoffe werden abfiltriert, zum Entfernen von anorganischen Stoffen gut mit Wasser gewaschen und aus Äthanol umkristalisiert. Man erhält γ-Chloracetoacet-p-toluidid; Smp. 159–160°C.

**Patentansprüche**

1. 4-Chlor-4-chlormethyloxetan-2-on.

2. Vefahren zur Herstellung von 4-Chlor-4-chlormethyloxetan-2-on nach Anspruch 1, dadurch gekennzeichnet, dass man Diketen mit Sulfurylchlorid unter Bedingungen umsetzt, bei denen freie Radikale gebildet werden.

3. Verfahren nach Anspruch 2, worin die freien Radikale durch ionisierende oder Ultraviolett-Bestrahlung des Reaktionsgemisches gebildet werden.

4. Verfahren nach Anspruch 2, worin die Umsetzung in Gegenwart einer zur Bildung von freien Radikalen befähigten chemischen Verbindung vorgenommen wird.

5. Verfahren nach Anspruch 2, dadurch gekennzeichnet, dass man die Umsetzung in Gegenwart eines organischen Peroxids, gegebenenfalls in Kombination mit Ultraviolett-Bestrahlung oder einem Metallionenkatalysator; einer anorganischen Peroxyverbindung, gegebenenfalls in Kombination mit einem Metallionenkatalysator, oder einer organischen Azoverbindung, gegebenenfalls in Kombination mit Ultraviolett-Bestrahlung, vornimmt.

6. Verfahren nach Anspruch 2, dadurch gekennzeichnet, dass man die Umsetzung in Gegenwart von tert-Butylperacetat, tert-Butylperbenzoat, Acetylperoxid, Benzoylperoxid, Di-isopropylperdicarbonat, Di-tert-butyl-cyclohexylperdicarbonat, Di-tert-butylperoxid oder tert-Butylhydroperoxid, vornimmt.

7. Verfahren nach Anspruch 2, dadurch gekennzeichnet, dass man die Umsetzung in Gegenwart von Wasserstoffperoxid oder Ammoniumpersulfat vornimmt.

8. Verfahren nach Anspruch 2, dadurch gekennzeichnet, dass man die Umsetzung in Gegenwart von Azobisisobutyronitril oder Azobisisopropan vornimmt.

9. Verfahren nach Anspruch 5, dadurch gekennzeichnet, dass man als Metallionenkatalysator Kupfer, Vanadium, Eisen oder Titan verwendet.

10. Verfahren nach Anspruch 2, dadurch gekennzeichnet, dass man die Umsetzung in Gegenwart von 0,1 bis 10 Mol.%, bezogen auf die eingesetzte Diketenmenge, einer zur Bildung freier Radikale befähigten Verbindung vornimmt.

11. Verfahren nach Anspruch 2, dadurch gekennzeichnet, dass man die Umsetzung in Gegenwart von 1 bis 5 Mol.%, bezogen auf die eingesetzte Diketenmenge, einer zur Bildung freier Radikale befähigten Verbindung vornimmt.

12. Verfahren nach Anspruch 2, dadurch gekennzeichnet, dass man die Umsetzung in Gegenwart eines unter den Reaktionsbedingungen inerten Lösungsmittels durchführt.

13. Verfahren nach Anspruch 2, dadurch gekennzeichnet, dass man die Umsetzung bei einer Temperatur zwischen 0 und 100°C durchführt.

## Claims

1. 4-Chloro-4-chlormethyloxetan-2-one.

2. A process for the preparation of 4-chloro-4-chloromethyloxetan-2-one according to claim 1, which comprises reacting diketene with sulphuryl chloride under conditions in which free radicals are formed.

3. A process according to claim 2, wherein the free radicals are formed by the application to the reaction mixture of ionising radiation or of ultraviolet radiation.

4. A process according to claim 2, wherein the reaction is effected in the presence of a chemical compound capable of forming free radicals.

5. A process according to claim 2, wherein the reaction is effected in the presence of an organic peroxide, optionally in conjunction with exposure to ultraviolet radiation or a metal ion catalyst; an inorganic peroxy compound, optionally in conjunction with a metal ion catalyst; or an organic azo compound, optionally in conjunction with exposure to ultraviolet radiation.

6. A process according to claim 2, wherein the reacting is effected in the presence of t-butyl peracetate, t-butyl perbenzoate, acetylperoxide, benzoylperoxide, di-isopropylperdicarbonate, di-t-butyl-cyclohexylperdicarbonate, di-t-butylperoxide or t-butylhydroperoxide.

7. A process according to claim 2 wherein the reaction is effected in the presence of hydrogen peroxide or ammonium persulphate.

8. A process according to claim 2, wherein the reaction is effected in the presence of azobisisobutyronitrile or azobisisopropane.

9. A process according to claim 5, wherein the metal ion catalyst is copper, vanadium, iron or titanium.

10. A process according to claim 2, wherein the reaction is effected in the presence of 0.1 to 10 mol.%, based on the amount used of diketene, of a compound capable of forming free radicals.

11. A process according to claim 2, wherein the reaction is effected in the presence of 1 to 5 mol.%, based on the amount used of diketene, of a compound capable of forming free radicals.

12. A process according to claim 2, wherein the reaction is effected in the presence of a solvent which is inert under the reaction conditions.

13. A process according to claim 2, wherein the reaction is effected at a temperature within the range from 0° to 100°C.

## Revendications

1. Chloro-4 chlorométhyl-4 oxétannone-2.

2. Procédé de préparation de la chloro-4 chlorométhyl-4 oxétannone-2 selon la revendication 1, procédé caractérisé en ce qu'on fait réagir le dicétène avec le chlorure de sulfuryle dans des conditions telles qu'il se forme des radicaux libres.

3. Procédé selon la revendication 2 selon lequel les radicaux libres sont formés par exposition du mélange réactionnel à un rayonnement ionisant ou à un rayonnement ultraviolet.

4. Procédé selon la revendication 2 selon lequel la réaction est effectuée en présence d'un composé chimique capable de former des radicaux libres.

5. Procédé selon la revendication 2 caractérisé en ce qu'on effectue la réaction en présence d'un peroxyde organique, éventuellement en association avec un rayonnement ultraviolet ou avec un catalyseur à base d'ions métalliques; d'un composé peroxydique minéral, éventuellement en association avec un catalyseur à base d'ions métalliques; ou d'un composé azoïque organique éventuellement en association avec un rayonnement ultraviolet.

6. Procédé selon la revendication 2 caractérisé en ce qu'on effectue la réaction en présence de peracétate de tert-butyle, de perbenzoate de tert-butyle, de peroxyde d'acétyle, de peroxyde de benzoyle, de perdicarbonate de di-isopropyle, de perdicarbonate de bis-(tert-butyl-cyclohexyle), de peroxyde de di-tert-butyle ou d'hydroperoxyde de tert-butyle.

7. Procédé selon la revendication 2 caractérisé en ce qu'on effectue la réaction en présence de peroxyde d'hydrogène ou de persulfate d'ammonium.

8. Procédé selon la revendication 2 caractérisé en ce qu'on effectue la réaction en présence d'azo-bis-isobutyronitrile ou d'azo-bis-isopropane.

9. Procédé selon la revendication 5 caractérisé en ce qu'on utilise, comme catalyseur à base d'ions métalliques, le cuivre, le vanadium, le fer ou le titane.

10. Procédé selon la revendication 2 caractérisé en ce qu'on effectue la réaction en présence de 0,1 à 10% en moles, par rapport à la quantité de dicétène mise en jeu, d'un composé capable de former des radicaux libres.

11. Procédé selon la revendication 2 caractérisé en ce qu'on effectue la réaction en présence de 1 à 5% en moles, par rapport à la quantité de dicétène mise en jeu, d'un composé capable de former des radicaux libres.

12. Procédé selon la revendication 2 caractérisé en ce qu'on effectue la réaction en présence d'un solvant inerte dans les conditions de la réaction.

13. Procédé selon la revendication 2 caractérisé en ce qu'on effectue la réaction à une température comprise entre 0 et 100°C.